# EUROPEAN PATENT APPLICATION

(11) **EP 0 741 142 A1**
(43) Date of publication of application: **06.11.1996**
(21) Application number: 94927104.3
(22) Date of filing: 27.09.1994
(51) Int. Cl.: C07K 14/46, A61K 38/17

(54) **PEPTIDE AND ANTITHROMBOTIC AGENT**

(30) Priority: 28.09.1993 JP 241666/93; 14.01.1994 JP 2691/94; 10.06.1994 JP 128518/94
(71) Applicant: Ajinomoto Co., Inc., Tokyo 104 (JP)
(72) Inventor: FUKUCHI, Naoyuki, Ajinomoto Co., Inc., Kawasaki-shi, Kanagawa 210 (JP); ISHII, Koichi, Ajinomoto Co., Inc., Kawasaki-shi, Kanagawa 210 (JP); KAIDA, Kenichi, Ajinomoto Co., Inc., Kawasaki-shi, Kanagawa 210 (JP); KOBAYASHI, Tsuyoshi, Ajinomoto Co., Inc., Kawasaki-shi, Kanagawa 210 (JP)
(74) Representative: Nicholls, Kathryn Margaret
(86) International application number: JP9401583
(87) International publication number: WO9509183

(57) **Abstract**

A peptide is obtained from a venom derived from *Vipera palestina* by screening various venoms for peptides that inhibit the binding of Von Willebrand factors with platelets even in a low concentration thereof and do not cause platelet aggregation. The obtained peptide has two differrent peptide chains and its molecular weight as determined by SDS polyacrylamide gel electrophoresis is about 25 kD and about 15 kD, respectively, under nonreducing and reducing conditions.

## Description

### Technical Field

The present invention relates to a peptide having an activity to inhibit binding of von Willebrand factor with platelets in blood, and an anti-thrombosis agent containing the peptide. In particular, the present invention relates to a peptide obtained from a snake venom originating from Vipera palestinae.

### Background Art

It is widely known that platelets closely participate in crisis of so-called thrombosis represented by myocardial infarction and cerebral thrombosis ("Platelets", edited by Yamanaka and Yamazaki, Igaku-Syoin, pp. 158-163 (1991)). Recently, it has been reported that the binding between von Willebrand factor as one of blood proteins and glycoprotein Ib located on platelet surfaces is important for platelets to adhere to intravascular subendotherial tissue, which is considered as an early reaction to cause thrombosis (J. P. Cean et al., J. Lab. Clin. Med., 87, 586-596 (1976)).

It is known that the binding between the two species of the proteins does not occur in an ordinary state, but it occurs only when a high shear stress is exerted in vivo (T. T. Vincent et al., Blood, 65, 823-831 (1985)). The methodology to observe the binding in vitro includes a widely spread method which uses certain substances such as ristocetin as an antibiotic (M. A. Howard, B. G. Firkin, Thromb. Haemostatis, 26, 362-369 (1971)) and botrocetin as a protein originating from a snake venom (M. S. Read et al., Proc. Natl. Acad. Sci. U.S.A., 75, 4514-4518 (1978)). Platelet aggregation occurs when these substances are added to a suspension of platelets. These aggregation depends on the binding between von Willebrand factor and Glycoprotein Ib (M. A. Howard, B. G. Firkin, M. S. Read et al., supra).

Several substances have been already reported, which exhibit an inhibiting action on the platelet aggregation described above. Such substances include, for example, Aurintricarboxylic acid (M. D. Phillips et al., Blood, 72, 1989-1903 (1988)). This substance has been reported to exhibit an anti-thrombosis property in an experiment with animals. The report describes that an anti-thrombosis action is obtained by inhibiting the binding of von Willebrand factor with platelets (J. Strony et al., Circulation, 81, 1106-1114 (1990)). Other substances which were reported to inhibit the binding between the both proteins include, for example, partial fragment peptides of von Willebrand factor or glycoprotein Ib (Y. Fujimura et al., J. Biol. Chem., 261, 381-385 (1986); K. Titani et al., Proc. Natl. Acad. Sci. U.S.A., 84, 5610-5614 (1987)).

It has been also reported that several peptides isolated from snake venoms have similar activities, as described in an international publication pamphlet of WO9208472 and a scientific paper written by Peng et al. (M. Peng et al., Blood, 81, 2321-2328 (1993)).

The international publication pamphlet of WO9208472 discloses peptides which inhibit the binding of von Willebrand factor with platelets, as obtained from snake venoms originating from Crotalus horridus horridus and Cerastes cerastes.

However, the pamphlet shows no data on any experiment with animals. Considering the short half-life of peptide substances in blood, it is assumed that those having higher activities in vitro provide higher efficacy in an experiment with animals. According to the international publication pamphlet of WO9208472, active peptides other than the aforementioned two species of the peptides obtained from snake venoms have been obtained on the basis of similar activities from Vipera russeli russeli and Pseudocerastes persicus. However, their specific activities are not clear. The pamphlet further describes that the activity to inhibit platelet aggregation is present in other several species of snake venoms. The activity to inhibit platelet aggregation in snake venoms has been already reported in many scientific papers (C-M. Teng, T-F. Hung, Platelet, 2, 77-87 (1991); B. H. Chao et al., Proc. Natl. Acad. Sci. U.S.A., 86, 8050-8054 (1989)), however, no evidence has been demonstrated for whether or not the inhibition on platelet aggregation exhibited by these toxins is mediated by the inhibition on binding of von Willebrand factor with platelets.

As for the peptide originating from Echis carinatus reported by Peng et al. (M. Peng et al., Blood, 81, 2321-2328 (1993)), we have obtained the following result as described in Example 2 in this specification. Namely, the inhibition on platelet aggregation occurred at a low concentration as mediated by the inhibition on binding of von Willebrand factor with platelets, however, a platelet aggregation-inducing action was observed at a high concentration. According to data described by Peng et al., their experiment with animals resulted in decrease in platelets. It is postulated that the decrease in platelets would be caused by, among other things, occurrence of thrombus formation triggered by the platelet aggregation-inducing action observed at a high concentration.

Besides, the reported peptides from snake venoms, which exhibit the activity to inhibit the binding of von Willebrand factor with platelets described above, several peptides, which exhibit similar molecular weights and have any action on platelets, have been already reported. It has been demonstrated that rattle snake lectin causes aggregation of platelets and erythrocytes (J. Hirabayashi et al., J. Biol. Chem., 266, 2320-2326 (1991)). It has been also demonstrated that botrocetin does not bind platelets, but it binds von Willebrand factor (Y. Fujimura et al., Biochemistry, 30, 1957-1964 (1991)). In another report, it has been demonstrated that alboaggregin obtained from a snake venom originating from Trimeresurus albolabris has an extremely strong platelet aggregation activity (M. Peng et al., Biochemistry, 30, 11529-11537 (1991)). According to recent studies, structures of these peptides have high homology in their amino acid sequences to that of the peptide originating from Crotalus horridus horridus (the international publication pamphlet of WO9208472) (J. Hirabayashi et al., J. Biol. Chem., 266, 2320-2326 (1991); E. Yoshida et al., Biochem. Biophys. Res. Commun., 191, 1386-1392 (1993); Y. Usami et al., Proc. Natl. Acad. Sci. U.S.A., 90, 928-932 (1993)).

According to the knowledge described above, it is postulated that peptides having high homology in their amino acid sequences to the aforementioned peptides are widely present in snake venoms. However, their actions may be various and diverse. Among a variety of such peptides, there may be a special peptide which inhibits the binding of von Willebrand factor with platelets at a lower concentration, and which does not exhibit the platelet aggregation-inducing action at a high concentration. It is an important task to discover such a special peptide, and the discovery of such a special peptide is essential in order to provide an anti-thrombosis drug having significant efficacy in vivo and characterized by inhibition on adhesion of platelets to subendothelial tissue.

The present invention has been made from a viewpoint as described above in order to obtain an efficacious agent as an anti-thrombosis drug, an object of which is to provide a peptide which inhibits the binding of von Willebrand factor with platelets at a low concentration, and which exhibits no platelet aggregation-inducing action.

### Disclosure of the Invention

In order to achieve the object described above, the present inventors selected several samples from snake venoms, the samples having an activity to strongly inhibit ristocetin-induced platelet aggregation and an activity to inhibit binding of von Willebrand factor to platelets, so that their active ingredients were investigate. As a result, it was revealed that extremely various peptides having various molecular weights had certain activities. Among them, active peptides were purified and isolated from three samples of snake venoms containing active peptides having molecular weights of about 25 kilodaltons to investigate their activities on platelets. Some obtained peptides belonged to a type of those which not only exhibited a strong activity to inhibit ristocetin-induced aggregation but also had an activity to induced platelet aggregation. However, it was found out that a peptide from Vipera palestinae did not induced platelet aggregation even at a high concentration. Thus the present invention has been completed.

Namely, the present invention lies in a peptide obtained from a snake venom of Vipera palestinae, the peptide having an activity to inhibit binding of von Willebrand factor to platelets. The present invention further provides an anti-thrombosis agent containing an efficacious component of the peptide and/or a pharmaceutically acceptable salt thereof.

The present invention will be described in detail below.

### 〈1〉 Peptide having activity to inhibit binding of von Willebrand factor with platelets

The peptide having the activity to inhibit the binding of von Willebrand factor to platelets according to the present invention (hereinafter referred to as "peptide of the present invention") is obtained from a snake venom of Vipera palestinae.

The snake venom of Vipera palestinae may be collected directly from Vipera palestinae. Alternatively, a commercially available snake venom of Vipera palestinae may be used. These venom sources may be subjected to purification by combination of various methods such as gel filtration, ion exchange, adsorption, and reverse phase column chromatography, affinity column chromatography, ultrafiltration, electrophoresis, and countercurrent distribution. Thus at least two species of peptides can be obtained, which inhibit the binding of von Willebrand factor with platelets.

Any of the peptides obtained as described above provides a molecular weight of about 25 kilodaltons in a non-reduced condition on SDS-polyacrylamide gel electrophoresis. On the other hand, in a reduced condition, for example, heated in the presence of 1 % mercaptoethanol, the peptide shows two different bands at positions of molecular weights of about 15 kilodaltons on SDS-polyacrylamide gel electrophoresis. According to this fact, the peptide of the present invention is a dimer comprising different peptide chains (α- and β-chains) of about 15 kilodaltons.

The structure of the peptide of the present invention can be determined as follows. For example, the peptide is subjected to reducing pyridylethylation, and the respective peptide chains are separated by using a method such as reverse phase high-performance liquid chromatography. The respective peptide chains, or peptide fragments digested by proteases such as lysylendopeptidase or by a chemical cleavage may be analyzed for their amino acid sequences by using an instrument such as amino acid sequence analyzer or an ordinary method for amino acid sequences analysis.

In accordance with the procedure described above, it has been found that the peptides of the present invention designated as VP-1 and VP-2 are novel peptides comprisinsg double strand peptides having N-terminal amino acid sequences shown in SEQ ID NOS. 1, 2 and SEQ ID NOS. 5 and 6, respectively, as illustrated in Examples described below.

VP-1 and VP-2 show the activity to inhibit ristocetin-induced platelet aggregation and the activity to inhibit the binding of von Willebrand factor to platelets. These activities are stronger than hitherto known peptides which do not show platelet aggregating activity even at a high concentration.

The N-terminal amino acid sequences of α- and β-chains of VP-1 are as shown in SEQ ID NOS. 1 and 2. A partial sequence of the α-chain including at least 32 amino acid residues from N-terminal, and a partial sequence of the β-chain including at least 30 amino acid residues from N-terminal are completely coincident with those of respective chains of VP-2. As for VP-2, an amino acid sequence from N-terminal to 52th amino acid residue of its α-chain, and an amino acid sequence from N-terminal to 43th amino acid residue of its β-chain are shown in SEQ ID NOS. 3 and 4 respectively. An entire amino acid sequence of the α-chain, and a major part of an amino acid sequence of the β-chain are shown in SEQ ID NOS. 5 and 6 respectively.

According to the fact described above, it is assumed that VP-1 and VP-2 have similar structures. However, because they can be separated from each other by ion exchange chromatography as illustrated in Examples, they are not an identical peptide. In any case, the peptide of the present invention is not limited to those having the sequences described above, because existence of certain peptides showing the inhibiting activity on the binding of von Willebrand factor to platelets are easily presumed to exist in respective individuals of Vipera palestinae. They are included in the scope of the present invention.

The knowledge described above has been revealed by the present invention for the first time. It is impossible to anticipate that the peptide of the present invention does not cause platelet aggregation even at a high concentration if any activity detected in unpurified snake venoms. Moreover, the peptide of the present invention inhibits the ristocetin-induced platelet aggregation and the binding of von Willebrand factor to platelet with higher specific activities than those of all peptides originating from snake venoms having been hitherto reported.

### 〈2〉 Pharmaceutical composition or agent of the present invention

The pharmaceutical composition or agent of the present invention utilizes the action possessed by the peptide of the present invention as described above, which is represented by an anti-thrombosis agent containing an active ingredient of the peptide of the present invention and/or a pharmaceutically acceptable salt thereof.

The peptide or the salt thereof may be used as a mixture of one or more species. In one embodiment, the agent may contain substances having any anti-thrombus function other than the peptide of the present invention. In such an embodiment, the peptide of the present invention is not necessarily a major component of the pharmaceutical composition or agent. The agent may be blended with other materials ordinarily used as components for drug preparation including, for example, proteins such as serum albumin, salts for buffering action or osmotic pressure adjustment, carriers, and excipients.

The type of the agent includes, for example, tablet, capsule, granule, syrup, suppository, ointment, injection, and instillation. The method of administration may be any of intravenous, subcutaneous, and oral administrations, as well as ophthalmic, transintestinal administrations, etc.

As for the dose upon administration to animals or human, an intended effect can be usually expected in a range of 0.1 µg/kg to 100 mg/kg, as an amount of the peptide of the present invention and/or the pharmaceutically acceptable salt thereof. Within this range, it is possible to select an amount with which the most excellent medicinal effect is obtained.

### Brief Description of the Drawings

Fig. 1 shows an ion exchange chromatogram of VP-1 and VP-2 by using a TSKgel-CM-5PW column. Obliquely hatched portions indicate VP-1 and VP-2 respectively.

Fig. 2 shows an ion exchange chromatogram of VP-1 by using TSKgel-DEAE-5PW.

Fig. 3 shows an ion exchange chromatogram of VP-2 by using TSKgel-DEAE-5PW.

Fig. 4 shows an ion exchange chromatogram of EC-1, EC-2, EC-3, and EC-4 by using TSKgel-CM-5PW.

Fig. 5 shows an ion exchange chromatogram of TA-3 by using TSKgel-CM-5PW.

Fig. 6 shows an ion exchange chromatogram of TA-1 and TA-2 by using TSKgel-CM-5PW.

Fig. 7 shows platelet aggregation-evoking activities of TA-1, TA-2, TA-3, EC-3, VP-1, and VP-2 respectively.

Fig. 8 shows an amino acid sequence and enzymatically digested fragments of α-chain of VP-2.

Fig. 9 shows an amino acid sequence and enzymatically digested fragments of β-chain of VP-2. Underlined portions have possibility of presence of further 1 to 3 amino acid residue(s).

Fig. 10 shows inhibition on ristocetin-induced aggregation by VP-1.

Fig. 11 shows inhibition on ristocetin-induced aggregation by VP-2.

Fig. 12 shows inhibiting activities of VP-1 and VP-2 on binding of von Willebrand factor to platelets.

### Best Mode for Carrying Out the Invention

Examples of the present invention will be described below.

### Example 1

### 〈1〉 Detection of inhibiting activity on binding of von Willebrand factor with platelets existing in snake venom

The inhibition on binding of von Willebrand factor with platelets was measured by an inhibition test based on a ristocetin-induced aggregation method as described 4in the following item (1), and an inhibition test for binding with platelets based on the use of radioisotope (¹²⁵I)-labeled von Willebrand factor as described in the following item (2).
(1) Fresh blood collected from healthy human added with 1/10 volume of 3.8 % sodium citrate was centrifuged at 900 rpm for 15 minutes to obtain human platelet rich plasma (PRP) to which equal volume of 0.15 M sodium chloride aqueous solution containing 20 mM phosphate buffer (pH 7.4) dissolved with 2 % paraformaldehyde was added, followed by being stored stationarily at 4 °C overnight to obtain fixed platelets. After the storage, platelets were recovered by centrifugation, and they were washed twice with a 0.15 M sodium chloride aqueous solution containing 20 mM phosphate buffer (pH 7.4). After the washing, the fixed platelets were suspended in the same solution and stored.
   A sample for measurement was added to the formalin-fixed platelet suspension thus prepared, to which human plasma (final concentration: 0.12 %) and ristocetin sulfate (produced by Sigma, final concentration: 0.5 mg/ml) were successively added. After shaking and agitation, the presence or absence of the inhibiting activity on platelet aggregation was observed macroscopically.
(2) An inhibition test for binding of ¹²⁵I-labeled von Willebrand factor with fixed platelets was performed in accordance with the method of Chopek et al. (M. W. Chopek et al., Biochemistry, 25, 3146-3155 (1986)). Namely, a suspension of fixed platelets prepared by the method of the item (1) described above was added with a sample for measurement, ristocetin sulfate (produced by Sigma), and ¹²⁵I-labeled von Willebrand factor, and reacted at room temperature for 30 minutes.

After that, the reaction solution was overlaid on a 20 % sucrose solution, and then a fixed platelet fraction was collected by centrifugation at 12,000 rpm for 5 minutes to measure the radioactivity by using a γ-counter (ARC301B, produced by Aloka). Thus the amount of bound von Willebrand factor was determined. Binding upon addition of an excessive amount of unlabeled von Willebrand factor was regarded as non-specific binding, while binding upon addition of no sample was regarded as maximum binding.

Based on the methods described above, the following seven samples, which were easily available and possessed the preferable inhibiting activity, were selected from commercially available lyophilized snake venom products (produced by Sigma) in order to specify the active substance.
(1) Vipera palestinae
(2) Echis carinatus
(3) Trimeresurus albolabris
(4) Trimeresurus flaboviridis
(5) Naja haje
(6) Naja nivea
(7) Crotarus admanteus

### 〈2〉 Measurement of molecular weight of active substance from snake venom

Each of the lyophilized snake venom products (10 mg) selected in the item 〈1〉 described above was dissolved in a 20 mM Tris-HCl buffer (pH 7.4, 1.5 ml) containing 0.9 % sodium chloride to separate components by using Sephadex G-75 gel filtration chromatography with the same buffer as a solvent (Sephadex G-75, produced by Pharmacia, column diameter: 26 mm, length: 90 mm). The flow rate was 0.5 ml/minute to fractionate and collect fractions (each 5 ml) by using fraction tubes.

The activity was measured by the same methods as described in the item 〈1〉. Table 1 shows elution positions (elution volumes) of the activity and estimated molecular weights.

**Table 1**

| Origin of snake venom | Elution position | Estimated approximate molecular weight |
|---|---|---|
| Vipera palestinae | 380-430 ml | 25,000 |
| Echis carinatus | 380-430 ml | 25,000 |
| Trimeresurus albolabris | 380-430 ml | 25,000 |
| | 300-340 ml | 50,000 |
| Trimeresurus flaboviridis | 260-300 ml | 100,000 |
| Naja haje | 230-280 ml | 100,000 or more |
| Naja nivea | 260-300 ml | 100,000 |
| Crotarus admanteus | 260-300 ml | 100,000 |

According to the result described above, it has been revealed that extremely various and diverse peptides are present, which exhibit inhibition on binding of von Willebrand factor with platelets, other than the hitherto reported peptides having the molecular weight of about 25 kilodaltons (international publication pamphlet of WO9208472; M. Peng et al., Blood, 81, 2321-2328 (1993)).

### 〈3〉 Isolation of active peptide from Vipera palestinae

A snake venom from Vipera palestinae (produced by Sigma, 50 mg) was dissolved in a 0.15 M sodium chloride aqueous solution (2.8 ml) containing 20 mM Tris-HCl buffer (pH 7.4). After that, insoluble matters were removed by centrifugation (15,000 rpm, 5 minutes) to perform gel filtration chromatography (column diameter: 2.6 cm, length 90 cm) by using Sephadex G-75 (produced by Pharmacia) with the same buffer as described above at a flow rate of 0.5 ml/minute.

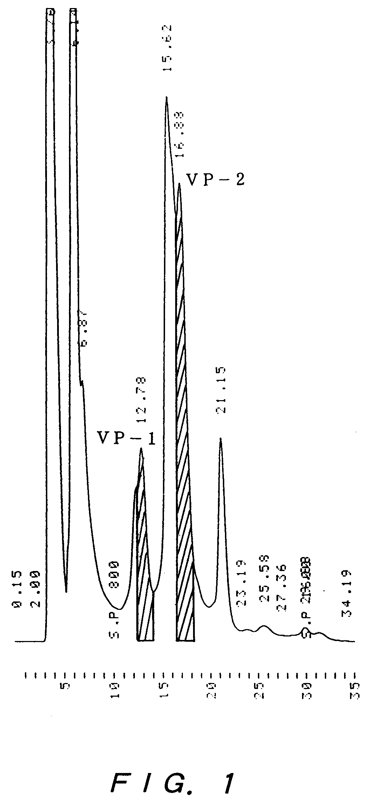

Each of fractions was measured for the inhibiting activity as described in the item 〈1〉. A fraction (elution volume: 390-430 ml) containing active substances was concentrated by ultrafiltration by using YM10 (produced by Amicon). After that, the solvent was substituted with a 50 mM ammonium acetate buffer (pH 4.5). An obtained concentrate was divided into two aliquots which were applied to ion exchange chromatography by using TSK-gel CM-5PW (produced by Tosoh, column diameter: 7.5 mm, length 75 mm) to fractionate and collect active peaks respectively. A peak having an earlier elution time was designated as VP-1, and a peak having a later elution time was designated as VP-2 (Fig. 1). An elution condition was as follows. Namely, two solvents (solvent A: 50 mM ammonium acetate buffer (pH 4.5), solvent B: 0.5 M ammonium acetate buffer (pH 6.4)) were used to perform elution with a linear concentration gradient so that the ratio (A:B) changed from (60:40) to (30:70) in 20 minutes.

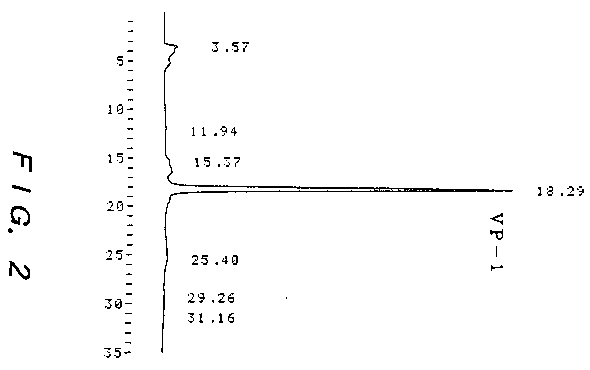

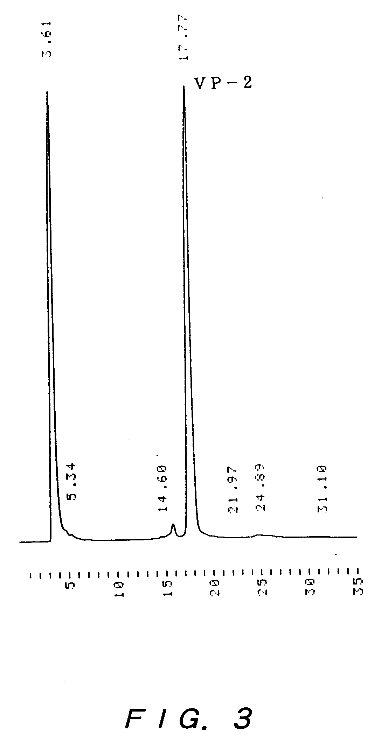

Fractions collected for respective samples were concentrated by using Centricon-10 (produced by Amicon), followed by further purification by ion exchange chromatography by using TSK-gel DEAE-5PW (produced by Tosoh, column diameter: 7.5 mm, length 75 mm). An elution condition was as follows. Namely, two solvents (solvent A: 50 mM ammonium acetate buffer (pH 9.0), solvent B: 0.5 M ammonium acetate buffer (pH 6.4)) were used to perform elution with a linear concentration gradient so that the ratio (A:B) changed from (100:0) to (50:50) in 20 minutes (Figs. 2 and 3).

VP-1 and VP-2 isolated as described above were finally concentrated by using Centricon-10 (produced by Amicon) respectively to provide physiological saline aqueous solutions thereof.

### Comparative Example 1: Isolation of Active Peptide from Echis carinatus

A snake venom from Echis carinatus (produced by Sigma, 25 mg) was dissolved in a 0.15 M sodium chloride aqueous solution (1 ml) containing 20 mM Tris-HCl buffer (pH 7.4). After that, insoluble matters were removed by centrifugation (15,000 rpm, 5 minutes) to perform gel filtration chromatography (column diameter: 2.6 cm, length 90 cm) by using Sephadex G-75 with the same buffer as that described above at a flow rate of 0.5 ml/minute.

The inhibition test for binding of von Willebrand factor with platelets was performed in the same manner as described in Example 1. An active fraction (elution volume: 390-410 ml) was concentrated by ultrafiltration by using Centriprep-10 (produced by Amicon). After that, the solvent was substituted with a 50 mM ammonium acetate buffer (pH 4.5). An obtained concentrate was divided into two aliquots which were applied to ion exchange chromatography by using TSK-gel CM-5PW (produced by Tosoh, column diameter: 7.5 mm, length 75 mm) to fractionate and collect active peaks respectively.

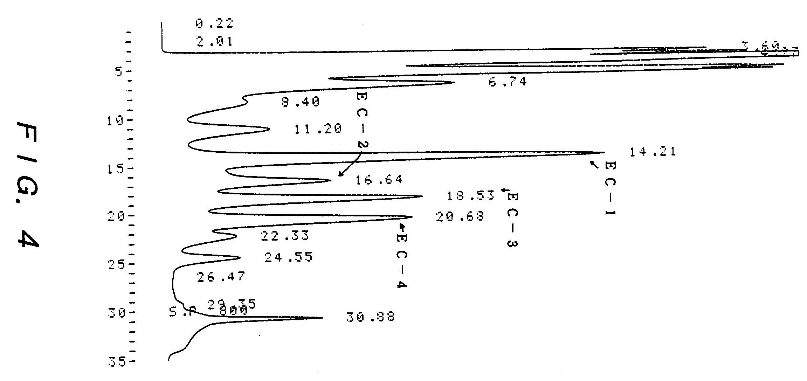

Four species of active peaks were present, which were designated as EC-1, EC-2, EC-3, and EC-4 as ranked from peaks having earlier elution times (Fig. 4). The four active fractions were concentrated by using Centricon-10 (produced by Amicon), followed by further purification by adsorption chromatography by using TSK-gel Phenyl-5PW (produced by Tosoh, column diameter: 4.6 mm, length: 75 mm) with an elution solvent of a sodium chloride linear concentration gradient (1.5 M to 0 M/20 minutes) containing 20 mM Tris-HCl buffer (pH 7.4) to obtain the four species as single active peptides respectively. One of the active peptides thus obtained was considered to be Echicetin reported by Peng et al. (M. Peng et al., Blood, 81, 2321-2328 (1993)) judging from its activity and behavior on the chromatography.

### Comparative Example 2: Isolation of Active Peptide from Trimeresurus albolabris

A snake venom from Trimeresurus albolabris (produced by Sigma, 50 mg) was dissolved in a 0.15 M sodium chloride aqueous solution (1.4 ml) containing 20 mM Tris-HCl buffer (pH 7.4). After that, insoluble matters were removed by centrifugation (15,000 rpm, 3 minutes) to perform gel filtration chromatography (column diameter: 2.6 cm, length 90 cm) by using Sephadex G-75 with the same buffer as that described above at a flow rate of 0.5 ml/minute.

The inhibition test for binding of von Willebrand factor to platelets was performed in the same manner as described in Example 1. Two active fractions (elution volumes: 300-350 ml and 390-440 ml) were concentrated by ultrafiltration by using Centriprep-10 (produced by Amicon). After that, the solvent was substituted with a 50 mM ammonium acetate buffer (pH 4.5).

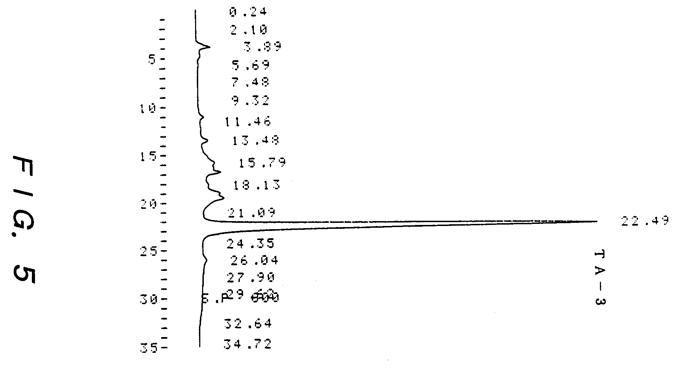

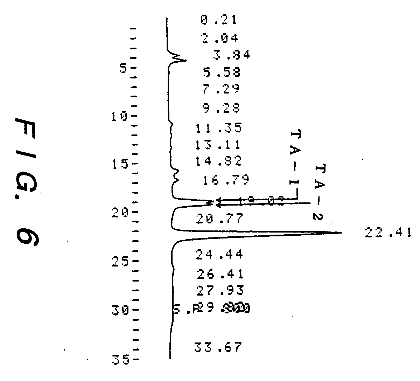

Concentrates of the respective active fractions were applied to ion exchange chromatography by using TSK-gel CM-5PW (produced by Tosoh, column diameter: 7.5 mm, length 75 mm) to obtain an active peptide TA-3 from the former active fraction (Fig. 5) and active peptides TA-1 and TA-2 from the latter active fraction (Fig. 6) as single peptides respectively. An elution condition was as follows. Namely, two solvents (solvent A: 50 mM ammonium acetate buffer (pH 4.5), solvent B: 0.5 M ammonium acetate buffer (pH 6.4)) were used to perform elution with a linear concentration gradient so that the ratio (A:B) changed from (100:0) to (0:100) in 20 minutes.

### Example 2: Properties of Active Peptide

### 〈1〉 Investigation on ristocetin-induced aggregation-inhibiting action and platelet aggregation-evoking activity of snake venom peptide

The active peptides isolated in Example 1 and Comparative Examples 1 and 2 were subjected to SDS-polyacrylamide gel electrophoresis in accordance with an ordinary method. As a result, as shown in Table 2, it was found that the respective active peptides comprised two different (or four different (TA-3)) peptide chains of about 14 to 17 kilodaltons in their molecular weights.

**Table 2**

| Name of peptide | Estimated approximate molecular weight | |
|---|---|---|
| | Non-reduced condition | Reduced condition |
| VP-1,-2 | 25,000 | 15,000, 15,000 |
| EC-1,-2,-3,-4 | 27,000 | 14,000, 15,000 |
| TA-1,-2 | 25,000 | 14,000, 15,000 |
| TA-3 | 46,000 | 14,000, 15,000, 15,000, 17,000 |

Any of the peptides described above inhibited the ristocetin-induced aggregation of fixed platelets described in Example 1 at a concentration of 5 µg/ml or less. Accordingly, we observed the action when these peptides were added to platelet rich plasma prepared by the same method as that described in Example 1. This measurment was performed with Hematracer-801 (produced by Niko Bioscience). The change in aggregation depending on passage of time was observed by introducing 100 µl of platelet rich plasma into a cuvette, agitating it at 37 °C for 3 minutes by using a stirrer bar, adding 11.1 µl of a peptide solution thereafter, and observing light transmittance.

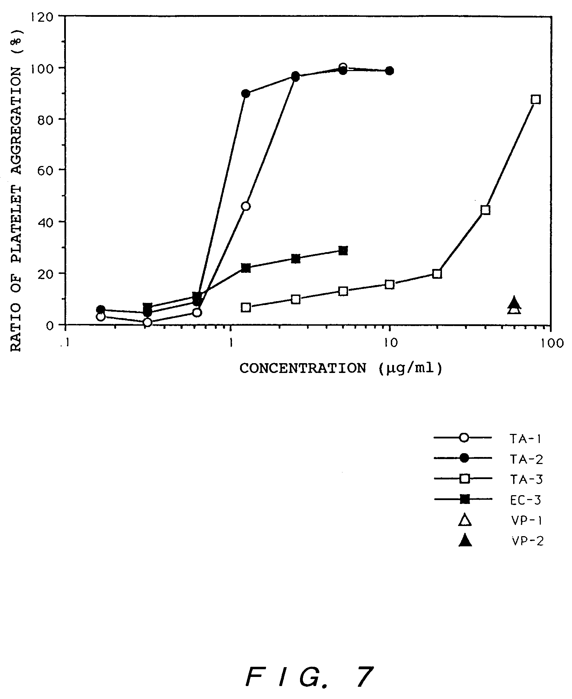

Fig. 7 shows the maximum aggregation ratio in 10 minutes versus the peptide concentration. As shown in Fig. 7, TA-1 and TA-2 exhibited extremely strong aggregating activities at low concentrations. TA-3, EC-1, EC-2, EC-3, and EC-4 exhibited platelet aggregation-evoking activities at higher concentrations (Fig. 7 shows only EC-3, however, EC-1, EC-2, and EC-4 had similar activities). On the contrary, VP-1 and VP-2 exhibited no platelet aggregation-evoking activity even at higher concentrations. According to these facts, it was revealed that these peptides, which are similar in their molecular weights and inhibiting activities on ristocetin-induced aggregation of fixed platelets, exhibit different behaviors in the platelet aggregation-evoking activity.

### 〈2〉 Structure of peptide of the present invention

It was found by isoelectric focusing that the active peptides VP-1 and VP-2 obtained from Vipera palestinae in accordance with the method described in Example 1 had pI values of 7.0 and 7.2 respectively. Further, an amino acid sequence of VP-2 was determined as follows. At first, VP-2 (100 µg) was dissolved in a 7 M guanidine hydrochloride aqueous solution (100 µl) containing 0.5 M Tris-HCl buffer (pH 8.5) and 10 mM ethylenediaminetetraacetic acid disodium salt (EDTA·Na2). After that, 4-vinylpyridine (1 µl) and tri-n-butylphosphine (2 µl) were added. A reaction was caused at room temperature overnight to perform reducing pyridylethylation.

The reaction solution described above was applied to liquid chromatography by using a column of TSKgel-Phenyl-5PW-RP (produced by Tosoh, diameter: 4.6 mm, length: 75 mm) to separate two peptide chains which constituted VP-2. Elution was performed at a flow rate of 1 ml/min with a linear concentration gradient (20 minutes) from an acetonitrile concentration of 31 % to a concentration of 52 % containing 0.1 % trifluoroacetic acid. A peptide chain having an earlier elution time was designated as VP-2 α-chain, and a peptide having a later elution time was designated as VP-2 β-chain. Each of them was lyophilized, and then dissolved in 30 % acetonitrile to perform amino acid sequence analysis from the amino terminal by using Protein Sequencer 470A (produced by Applied Biosystems).

The reducing pyridylethylated peptide obtained as described above was dissolved in a 7 M guanidine hydrochloride aqueous solution (25 µl) containing 0.5 M Tris-HCl (pH 8.5) and 10 mM ethylenediaminetetraacetic acid disodium salt, followed by being stored at 37 °C for 1 hour. After that, a digestion reaction with lysylendopeptidase (produced by Wako Pure Chemical, 2 µg) was performed at 37 °C for 2 hours in a reaction solution having final concentrations of 2 M guanidine hydrochloride, 0.1 M Tris-HCl buffer (pH 8.5), and 2 mM ethylenediaminetetraacetic acid disodium salt.

Digested fragments obtained by the digestion reaction described above were separated, fractionated, and collected by high-performance liquid chromatography by using a reverse phase column (SSC-VP-318, produced by Senshu Kagaku, diameter: 4.6 mm, length: 250 mm) to analyze respective amino acid sequences by using Protein Sequencer 470A (produced by Applied Biosystems).

Additionally, the peptides were fragmented with proteinase Glu-C (produced by Boehringer Mannheim), and their amino acid sequences were analyzed, in accordance with methods similar to those described above.

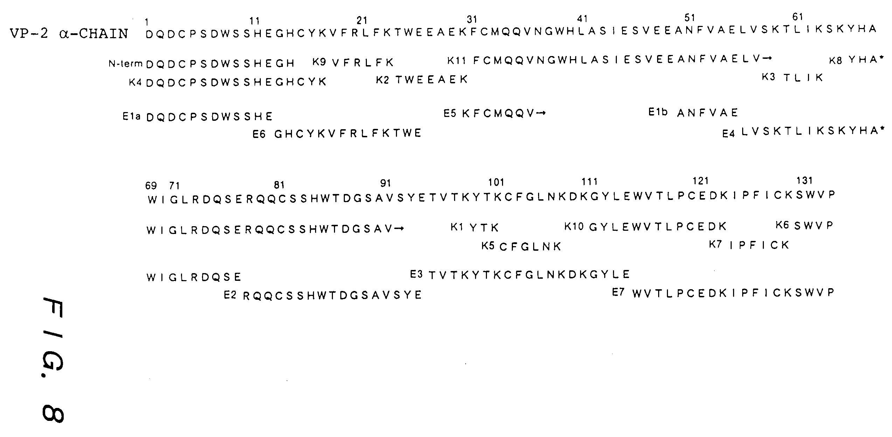

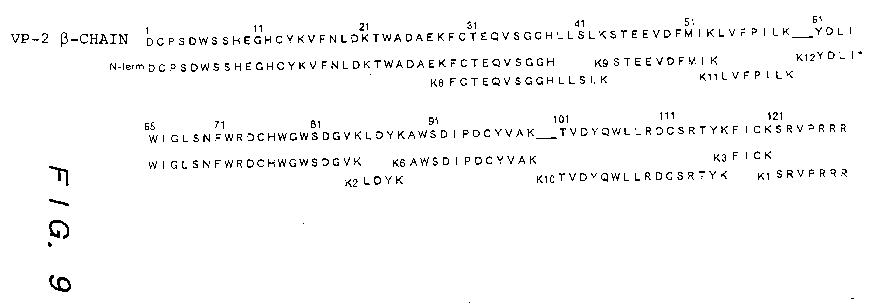

The amino acid sequence of the VP-2 α-chain is shown in SEQ ID NO. 5, and the amino acid sequence of the VP-2 β-chain is shown in SEQ ID NO: 6, as clarified as described above. Fig. 8 shows enzymatically digested fragments and their amino acid sequences of the VP-2 α-chain, and Fig. 9 shows enzymatically digested fragments and their amino acid sequences of the VP-2 β-chain. In Figs. 8 and 9, K indicates parts of fragments digested with lysylendopeptidase, and E indicates parts of fragments digested with proteinase Glu-C.

It is noted that the accuracy of ordinarily available amino acid sequencing methods is not complete. Accordingly, mistakes in reading such as deletion of amino acid residues and confusion with other amino acid residues are apt to occur to some extent. It is difficult to deny the possibility that such mistakes in reading may be included in the amino acid sequences described in this specification. However, even if such mistakes in reading are included, the sequences shown in SEQ ID NOS. 3 to 6 are considered to be sufficient to specify the peptides of the present invention in combination with their properties such as physiological activities and molecular weights.

SEQ ID NO: 3 shows a partial sequence (52 amino acid residues from N-terminal) which is conceived to have higher assurance in the amino acid sequence of the VP-2 α-chain.

On the other hand, the VP-2 β-chain has a possibility that 1 to 3 unidentified amino acid(s) may be present between 60th lysine and 61th tyrosine in SEQ ID NO: 6. It also has a possibility that 1 to 3 unidentified amino acid(s) may be present between 100th lysine and 101th threonine. SEQ ID NO: 4 shows a partial sequence (43 amino acid residues from N-terminal) which is conceived to have higher assurance in the amino acid sequence of the VP-2 β-chain.

VP-1 was also analyzed for its amino terminal amino acid sequence after reducing pyridylethylation in the same manner as described above. Results are shown in SEQ ID NOS: 1 and 2. According to the results, it was found that VP-1 had the same amino acid sequence as that of VP-2 at least at its N-terminal portion.

Further, peptides originating from the respective chains of VP-1 obtained by reducing pyridylethylation were digested with lysylendopeptidase. Obtained digested fragments were compared with the digested fragments originating from VP-2 with respect to their elution positions in reverse phase high-performance liquid chromatography. As a result, a difference was found in one fragment originating from the α-chain. As a result of amino acid sequence analysis, it was found that 110th glycine and 122th lysine of VP-2 were substituted with glutamic acid and aspartic acid in VP-1 respectively.

### 〈3〉 Activity of peptide of the present invention on platelets

The inhibiting activity on various types of aggregation of platelet rich plasma was measured for VP-1 and VP-2 obtained from Vipera palestinae by using Hematracer-801 (produced by Niko Bioscience) in the same manner as the method described in the item 〈1〉. Ristocetin (final concentration: 1.2 mg/ml), botrocetin (1 µg/ml), ADP (3 µM), and collagen (10 µg/ml) were used as agglutinogens. The ratio of inhibition on aggregation, as compared with a control group with no sample addition, was measured by adding each peptide to the platelet rich plasma, agitating them at 37 °C for 3 minutes, and adding the agglutinogen described above.

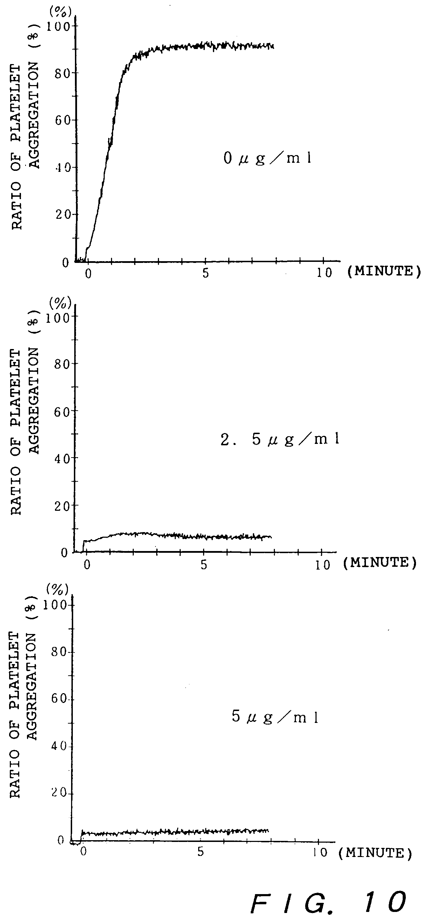

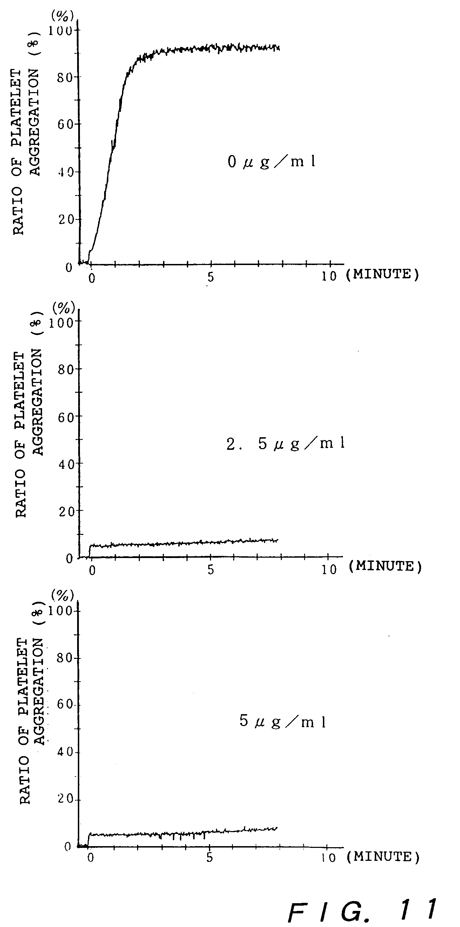

Figs. 10 and 11 show inhibiting activities of VP-1 and VP-2 on ristocetin-induced aggregation, among obtained results of the measurement. It was found that both VP-1 and VP-2 exhibited inhibition of 90 % or more on the ristocetin-induced aggregation at 2.5 µg/ml, and they had inhibiting activities stronger than that of a peptide CHH-B originating from Crotalus horridus horridus described in the international publication pamphlet of WO9208472 (inhibition of about 30 % at 3 µg/ml). The both peptides also exhibited inhibiting activities on the botrocetin-induced aggregation at an approximately similar concentration. On the other hand, both VP-1 and VP-2 exhibited no inhibition on the ADP-or collagen-induced aggregation even at a concentration of 20 µg/ml. Accordingly, it has been demonstrated that the peptides of the present invention specifically inhibit aggregation depending on the binding between von Willebrand factor and glycoprotein Ib such as ristocetin-induced aggregation and botrocetin-induced aggregation.

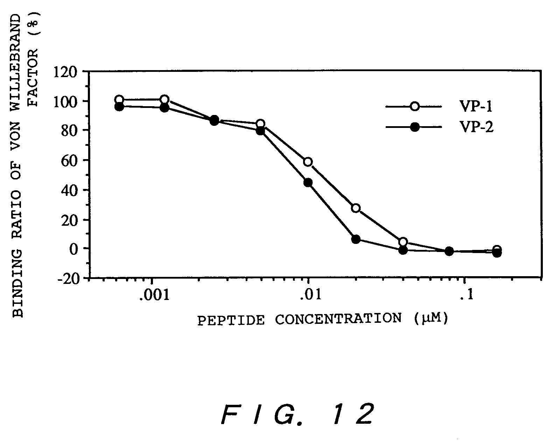

Further, in order to clarify the inhibiting action of the two proteins described above on the binding, the method described in Example 1 was used to measure inhibition on the binding of ¹²⁵I-labeled von Willebrand factor with platelets. As a result, both VP-1 and VP-2 inhibited the binding of von Willebrand factor with platelets depending on their concentrations as shown in Fig. 12. The inhibition concentration for 50 % binding (IC₅₀) was 0.008 to 0.016 µM for the both peptides. Accordingly, it has been demonstrated that the peptides of the present invention inhibit the binding between von Willebrand factor and glycoprotein Ib at a lower concentration than that of CHH-B described in the international publication pamphlet of WO9208472 (IC₅₀: 0.1 to 0.2 µM).

### Industrial Applicability

The peptide obtained in accordance with the present invention inhibits binding of von Willebrand factor to platelets, the binding closely participating in crisis of thrombosis. This activity is expressed at a concentration lower than those of conventionally known substances. Further, the peptide does not induce platelet aggregation even at a high concentration. Accordingly, the peptide is useful as an anti-thrombosis drug.

## Claims

1. A peptide obtained from a snake venom of Vipera palestinae, the peptide having an activity to inhibit binding of von Willebrand factor with platelets.

2. A peptide according to claim 1, wherein said peptide comprises two different peptide chains, said peptide having a molecular weight of about 25 kilodaltons under a non-reduced condition, or about 15 kilodaltons under a reduced condition on SDS-polyacrylamide gel electrophoresis.

3. A peptide according to claim 2, wherein one of said two peptide chains has an amino acid sequence shown in SEQ ID NO: 1 in Sequence Listing at its N-terminal, and the other has an amino acid sequence shown in SEQ ID NO: 2 at its N-terminal.

4. A peptide according to claim 2, wherein one of said two peptide chains has an amino acid sequence shown in SEQ ID NO: 3 in Sequence Listing at its N-terminal, and the other has an amino acid sequence shown in SEQ ID NO: 4 at its N-terminal.

5. A peptide according to claim 2, wherein one of said two peptide chains has an amino acid sequence shown in SEQ ID NO: 5 in Sequence Listing, and the other has an amino acid sequence shown in SEQ ID NO: 6.

6. An anti-thrombosis agent containing an efficacious component of the peptide as defined in any one of claims 1 to 5 and/or a pharmaceutically acceptable salt thereof.


SEQUENCE LISTING

(1) GENERAL INFORMATION:
(i) APPLICANT: AJINOMOTO CO., INC.
(ii) TITLE OF INVENTION: PEPTIDE AND ANTI-THROMBOSIS AGENT
(iii) NUMBER OF SEQUENCES: 6
(iv) CORRESPONDENCE ADDRESS:
(A) ADDRESSEE:
(B) STREET:
(C) CITY:
(E) COUNTRY:
(F) ZIP:
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: FastSEQ Version 1.5
(vi) CURRENT APPLICATION DATA:
(A) APPLICATION NUMBER:
(B) FILING DATE:
(C) CLASSIFICATION:
(vii) PRIOR APPLICATION DATA:
(A) APPLICATION NUMBER:
(B) FILING DATE:
(viii) ATTORNEY/AGENT INFORMATION:
(A) NAME:
(B) REGISTRATION NUMBER:
(ix) TELECOMMUNICATION INFORMATION:
(A) TELEPHONE:
(B) TELEFAX:

(2) INFORMATION FOR SEQ ID NO:1:
(i) SEQUENCE CHARACTERISTICS:
(A) LENGTH: 32 amino acids
(B) TYPE: amino acid
(D) TOPOLOGY: linear
(ii) MOLECULE TYPE: peptide
(xi) SEQUENCE DESCRIPTION: SEQ ID NO:1:
Asp Gln Asp Cys Pro Ser Asp Trp Ser Ser His Glu Gly His Cys Tyr
1               5                   10                  15
Lys Val Phe Arg Leu Phe Lys Thr Trp Glu Glu Ala Glu Lys Phe Cys
            20                  25                  30

(2) INFORMATION FOR SEQ ID NO:2:
(i) SEQUENCE CHARACTERISTICS:

(A) LENGTH: 30 amino acids
(B) TYPE: amino acid
(D) TOPOLOGY: linear
(ii) MOLECULE TYPE: peptide
(xi) SEQUENCE DESCRIPTION: SEQ ID NO:2:
Asp Cys Pro Ser Asp Trp Ser Ser His Glu Gly His Cys Tyr Lys Val
1               5                   10                  15
Phe Asn Leu Asp Lys Thr Trp Ala Asp Ala Glu Lys Phe Cys
            20                  25                  30

(2) INFORMATION FOR SEQ ID NO:3:
(i) SEQUENCE CHARACTERISTICS:
(A) LENGTH: 52 amino acids
(B) TYPE: amino acid
(D) TOPOLOGY: linear
(ii) MOLECULE TYPE: peptide
(xi) SEQUENCE DESCRIPTION: SEQ ID NO:3:
Asp Gln Asp Cys Pro Ser Asp Trp Ser Ser His Glu Gly His Cys Tyr
1               5                   10                  15
Lys Val Phe Arg Leu Phe Lys Thr Trp Glu Glu Ala Glu Lys Phe Cys
            20                  25                  30
Met Gln Gln Val Asn Gly Trp His Leu Ala Ser Ile Glu Ser Val Glu
        35                  40                  45
Glu Ala Asn Phe
    50

(2) INFORMATION FOR SEQ ID NO:4:
(i) SEQUENCE CHARACTERISTICS:
(A) LENGTH: 43 amino acids
(B) TYPE: amino acid
(D) TOPOLOGY: linear
(ii) MOLECULE TYPE: peptide
(xi) SEQUENCE DESCRIPTION: SEQ ID NO:4:
Asp Cys Pro Ser Asp Trp Ser Ser His Glu Gly His Cys Tyr Lys Val
1               5                   10                  15
Phe Asn Leu Asp Lys Thr Trp Ala Asp Ala Glu Lys Phe Cys Thr Glu
            20                  25                  30
Gln Val Ser Gly Gly His Leu Leu Ser Leu Lys
        35                  40

(2) INFORMATION FOR SEQ ID NO:5:
(i) SEQUENCE CHARACTERISTICS:
(A) LENGTH: 132 amino acids
(B) TYPE: amino acid
(D) TOPOLOGY: linear

(ii) MOLECULE TYPE: peptide
(xi) SEQUENCE DESCRIPTION: SEQ ID NO:5:
Asp Gln Asp Cys Pro Ser Asp Trp Ser Ser His Glu Gly His Cys Tyr
1               5                   10                  15
Lys Val Phe Arg Leu Phe Lys Thr Trp Glu Glu Ala Glu Lys Phe Cys
            20                  25                  30
Met Gln Gln Val Asn Gly Trp His Leu Ala Ser Ile Glu Ser Val Glu
        35                  40                  45
Glu Ala Asn Phe Val Ala Glu Leu Val Ser Lys Thr Leu Ile Lys Ser
    50                  55                  60
Lys Tyr His Ala Trp Ile Gly Leu Arg Asp Gln Ser Glu Arg Gln Gln
65                  70                  75                  80
Cys Ser Ser His Trp Thr Asp Gly Ser Ala Val Ser Tyr Glu Thr Val
                85                  90                  95
Thr Lys Tyr Thr Lys Cys Phe Gly Leu Asn Lys Asp Lys Gly Tyr Leu
            100                 105                 110
Glu Trp Val Thr Leu Pro Cys Glu Asp Lys Ile Pro Phe Ile Cys Lys
        115                 120                 125
Ser Trp Val Pro
    130

(2) INFORMATION FOR SEQ ID NO:6:
(i) SEQUENCE CHARACTERISTICS:
(A) LENGTH: 127 amino acids
(B) TYPE: amino acid
(D) TOPOLOGY: linear
(ii) MOLECULE TYPE: peptide
(xi) SEQUENCE DESCRIPTION: SEQ ID NO:6:
Asp Cys Pro Ser Asp Trp Ser Ser His Glu Gly His Cys Tyr Lys Val
1               5                   10                  15
Phe Asn Leu Asp Lys Thr Trp Ala Asp Ala Glu Lys Phe Cys Thr Glu
            20                  25                  30
Gln Val Ser Gly Gly His Leu Leu Ser Leu Lys Ser Thr Glu Glu Val
        35                  40                  45
Asp Phe Met Ile Lys Leu Val Phe Pro Ile Leu Lys Tyr Asp Leu Ile
    50                  55                  60
Trp Ile Gly Leu Ser Asn Phe Trp Arg Asp Cys His Trp Gly Trp Ser
65                  70                  75                  80
Asp Gly Val Lys Leu Asp Tyr Lys Ala Trp Ser Asp Ile Pro Asp Cys
                85                  90                  95
Tyr Val Ala Lys Thr Val Asp Tyr Gln Trp Leu Leu Arg Asp Cys Ser
            100                 105                 110
Arg Thr Tyr Lys Phe Ile Cys Lys Ser Arg Val Pro Arg Arg Arg
        115                 120                 125